# EUROPEAN PATENT APPLICATION

(11) **EP 1 123 706 A1**
(43) Date of publication of application: **16.08.2001**
(21) Application number: 00200412.5
(22) Date of filing: 08.02.2000
(51) Int. Cl.: A61K 35/30, C12N 5/06

(54) **Neuronal cell division**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: van Noort, Johannes Maria, 2332 AT Leiden (NL); Bajramovic, Jeffrey John, 91330 Yerresen (FR); Bsibsi, Malika, 2324 VK Leiden (NL)
(74) Representative: Ottevangers, Sietse Ulbe

(57) **Abstract**

The present invention relates to a composition or factor which stimulates neurons, preferably human neurons, to cell division. The composition or factor can be recovered from white matter of brain, for instance in the form of an extract. The present composition or factor can be employed for the in vitro production of neurons, or as a component of pharmaceutical preparations for the treatment of brain disorders involving disturbed neuronal cell division.

## Description

This invention relates to a composition or factor which stimulates neurons (in particular of mammalian, preferably human, origin) to divide, and to a method for obtaining such a composition or factor.

Neurodegenerative disorders constitute a considerable health problem with very great socioeconomic consequences. In particular in the more developed parts of the world, where life expectancy is comparatively high, a considerable part of the population are afflicted by various brain disorders, among which diseases such as Alzheimer's disease , Parkinson's disease, Huntington's disease and multiple sclerosis. Brain or spinal cord injury caused by trauma also presents society with considerable problems.

An important factor in the normally irreversible development of brain injury and brain disorders is the fact that adult human neurons, normally speaking, do not proceed to divide (O'Leary, 1993; Zhu et al, 1999). Nor, under normal conditions, does regeneration of damaged neuronal processes, axons, take place. As a consequence, neuronal damage due to encephalopathy as a rule remains present for life and is not repaired. The inability of adult neurons to divide is generally regarded as an almost dogmatic fact. Therefore, regeneration of neuronal tissue as therapy for brain disorders or in the treatment of brain or spinal cord injury has long been regarded as an unrealistic option. Also more fundamental studies of the characteristics of purified adult neurons in culture are very difficult because after isolation the cells as a rule die off soon. Only when adult neurons in the form of brain slices are taken into culture, surrounded by other supporting cells in the central nervous system, can they be kept alive for a number of weeks.

Already many attempts have been made to maintain adult neurons in culture and/or to cause them to divide. Neuronal cells that do come to division in culture are not yet completely developed embryonic or neonatal neurons, or neuronal precursor cells. This last type of cell still occurs in the adult central nervous system (Reynolds and Weiss, 1992; Richards et al, 1992; Lois and Alvarez-Buylla, 1994; Gage et al, 1995). Recently, much research on these neuronal precursor cells (variously called precursor neurons, progenitor neurons or neuronal stem cells) has been initiated. It is rapidly becoming clear that the adult central nervous system still contains surprisingly large numbers of such precursor cells. In learning processes, upon damage, and under the influence of factors affecting hormonal balance, these precursor neurons can migrate from particular areas of the nervous system, such as the subventrical zone or the hippocampus, to, for instance, the neocortical regions and grow out to be full-grade neurons. This phenomenon yields a plasticity in the adult central nervous system which has recently been found to be much greater than was previously suspected (Eriksson et al, 1998; Kornack & Rakic, 1999; Gould et al, 1999; Vescovi & Snyder, 1999). In particular in the hippocampus, there are large numbers of neuronal precursor cells that in culture exhibit good properties with regard to regeneration and growth (Brewer, 1997, 1999; Ray et al, 1993; Walicke et al, 1986; Whitson et al, 1989). In other areas, too, including the striatum, the neocortex and in the optical nerves, neuronal precursor cells have been found (Palmer et al, 1999; De Lima & Voigt, 1999). Neuronal precursor cells can develop not only into neurons but also into astrocytes (supporting cells in the central nervous system) (Reynolds & Weiss, 1992).

Growth and differentiation of neuronal precursor cells under culture conditions as a rule require special growth factors. Growth factors such as fibroblast growth factor-2 (FGF-2), transforming growth factor-b1 (TGF-b1), epidermal growth factor (EGF), amyloid-b protein, interferon-gamma or nerve growth factor (NGF) belong to the series of growth factors which are known to have an effect under culture conditions on the growth of precursor neurons (Barish et al, 1991; Cataneo et al, 1990; Kirschenbaum and Goldman, 1995; Morrison et al, 1986, 1987; Unsicker et al, 1987; Walicke et al, 1986; Whitson et al, 1989). The direction in which a neuronal precursor cells can develop in vitro (into astrocytes or into neurons) is strongly influenced by a number of these growth factors and in particular by EGF and FGF-2 (Reynolds & Weiss, 1992; Kuhn et al, 1997; Kalyani et al, 1998). The development of neuronal precursor cells into actual neurons seems to be controlled mainly by FGF-2 (Palmer et al, 1999).

Regeneration of damaged neurons can occur not only through division, but also through renewed growth of the neuron processes (dendrites or axons). Some ten years ago, it was discovered that the regeneration of neuronal dendrites under normal conditions is inhibited by certain components in the myelin sheath in the white matter (the myelin sheath or myelin is the protective membrane around axons, the neuronal processes) (Schnell & Schwab, 1990; Schwab, 1996). Various myelin components that have this effect have meanwhile been identified. Neutralization of their inhibitory action by addition of specific antibodies can lead to accelerated regeneration of neuronal dendrites and axons (Niederösch et al, 1999).

While certain components in the white matter have thus been identified as substances having a certain capacity towards de-differentiation in neurons, there are no reports of cell division in neurons under the influence of these components, or under the influence of any other substances. Therefore, when using the term "neuronal regeneration" such as it regularly crops up in the literature, a proper distinction must be made between regeneration in the sense of partial repair of the process structure, and actual cell division.

Cell division and differentiation of precursor neurons is a phenomenon which has been well described by now. It has become clear that this cell division, in any case under culture conditions, as a rule requires the use of serum-free medium as well as the presence of certain growth factors such as FGF-2. Bringing effectively differentiated adult neurons to cell division has not been described yet.

Presently, surprisingly, a factor has been discovered which stimulates human neurons to divide. This cell division has been observed inter alia in vitro in neurons from the adult cerebral cortex. The factor, which consists of one substance or a combination of different substances, is contained in a water soluble extract of human white matter from the brains. When added in small amounts to primary cultures of adult human cortical neurons, the extract leads to multiplication of the neurons. After addition of the extract, within 24 hours the number of viable neurons in culture doubled. By regularly repeating the stimulation, neurons in culture can be proliferated and maintained alive over a period of at least 60 days. In this period, further, limited regeneration of neuronal dendritic processes takes place.

Thus, the invention relates to a method for obtaining a composition or factor which is capable of stimulating neurons to cell division, in which such a composition or factor is recovered from white matter of the brain or tissue of the central nervous system.

More particularly, the invention provides a method for preparing an extract comprising a factor which induces cell division of neurons, wherein a sample is taken of white matter from brains or from tissue of a central nervous system, which sample is homogenized and centrifuged, thereby yielding the extract contemplated.

The brain from which the sample is taken can in principle originate from any type of organism that has such tissue. The extract that is obtained will be able to stimulate neurons of the same type of organism to cell division. Preferably, the organism is a mammal, more preferably a human.

Brain tissue, as well as tissue coming from the central nervous system, typically consists of two kinds of tissue, designated by gray matter and white matter. The sample that is taken in accordance with the invention comes from the white matter. It can be distinguished from the gray matter by color. The term white matter designates those parts of the central nervous system that are characterized by the presence of a relatively large amount of myelin. The anatomical position of this in the brain and the central nervous system is generally known.

If desired, the sample can be stored for some time at a strongly reduced temperature, preferably below -60°C. The entire method for preparing the extract is preferably carried out at a temperature lower than 10°C, more preferably lower than 5°C.

The sample is included in a suitable buffer. An example of a suitable buffer is a phosphate-buffered physiological salt solution. The pH of the buffer is preferably approximately neutral, i.e. between 6.5 and 8.5, more preferably between 7 and 8. The ratio between sample and buffer will typically be between 1:3 and 1:5, based on weight.

The suspension thus obtained is homogenized. This can be done in any known manner, for instance by stirring well, using a so-called Dounce homogenizer, or by grinding, for instance with a mortar and pestle.

The homogenized suspension is subsequently centrifuged. Preferably, centrifugation is done so that the greater part of mass present that is not soluble in water is separated from mass that is soluble in water. Typically, centrifugation will take a period of at least 15 minutes.

After centrifugation, a solid portion and a supernatant are obtained, which are separated from each other. If desired, the yield can be increased by including the solid substance in the above-described buffer once again, homogenizing, and centrifuging again.

Thus the extract contemplated is obtained, which comprises a factor capable of stimulating cell division of neurons. The invention encompasses the extract thus obtainable, as well as the factor stimulating neuronal cell division that is present in such extract, regardless of whether the factor is obtained though isolation from the extract, through synthesis or in any other way.

In a preferred embodiment, the supernatant or the combined supernatants are subsequently dialyzed against a suitable buffer. Preferably, the same buffer is used here as that in which the sample of the white matter of the brains was included. During dialysis, the conditions are preferably selected such that substances of a molecular weight of less than 3,000 are removed.

A factor according to the invention, or a combination of such factors, for instance in the form of an extract, can be utilized in in vitro studies and the production of human neurons. For a proper study of defined cell types, the possibility of carrying out in vitro studies with purified cell populations is very important. At the moment, much of the neurobiological, cell biological and immunological functions of neurons is still unknown because isolated neurons in culture cannot be grown sufficiently long or with sufficient maintenance of viability. A factor which stimulates the division of neurons and greatly improves the viability of the cells under culture conditions brings a change to this. The simple fact that, using the invention, neurons can studied in vitro much better is an important improvement of the possibilities of carrying out fundamental and applied research on neurons. In addition, the invention constitutes a basis for proceeding to the production of neurons, for instance for transplantation purposes or for vaccine production. (Various viruses capable of infecting human neurons do not do so, or do so only with difficulty, in other cell types. For the production of virus preparations, as is required for the preparation of vaccines, use can be made only of the cell type in which the virus thrives. If these are neurons, the invention can find application in this area.)

A factor according to the invention or an extract comprising that factor can further be used in the identification of molecular signals for neuronal cell division. Only little is known about the biochemical processes stimulating neurons to growth and/or differentiation. The invention can be used to identify both receptors on the surface of neurons and intracellular signal transduction routes which are respectively occupied and activated by the factor and which apparently exert growth-stimulating action in neurons. If such receptors and/or signal transduction routes can be identified, this also clears the road for screening other substances (e.g. series of pharmaceutical preparations) with which these same signals can be induced. Thus the invention can lead to the identification of other substances, which have the same biological effect but are possibly easier to produce or to use than a white matter extract or the active substances therein.

Further, the present factor or an extract comprising the factor can find application in therapy and diagnostics in brain disorders. The finding of one or more factors that stimulate human neurons to divide can be used in situations where proliferation and/or regeneration of neurons in vivo can be expected to have a therapeutic effect. Broadly, this is the case with all brain disorders where neurons degenerate, die off or otherwise deteriorate in their function, or have been or are being damaged. To that end, the factor could be administered in vivo in such a manner that neurons regenerate and/or proceed to divide in a controlled manner, as they do in vitro.

Thus, the invention further relates to a pharmaceutical preparation which comprises the present factor, optionally in combination with a suitable carrier. This preparation can be administered by way of the bloodstream (intravenously), by way of the mouth (orally), by injection into the muscles (intramuscularly), by way of the skin (epidermically) or directly by injection into the central nervous system (intracranially). Preferably, the preparation is administered intracranially. Examples of disorders where regeneration of neurons in accordance with the invention can mediate a therapeutic effect include inter alia Alzheimer's disease and other forms of dementia, Huntington's disease, Parkinson's disease, multiple sclerosis, amyotrophic lateral sclerosis, stroke (cerebral hemorrhage) and mechanical damage due to, for instance, spinal cord lesion.

Knowledge of a factor or of factors that stimulate neurons to divide can lead to improved methods of diagnostics (and treatment) of brain disorders where a spontaneously arising transition of quiescent adult neurons to the mitotic phase of the cell cycle contributes to the disease. This is possibly the case, for instance, in Alzheimer's disease (Zhu et al, 1999). There are indications that in this disease a spontaneously arising but incomplete transition of quiescent neurons to the mitotic phase is part of the pathology. Identification of the same receptors or intracellular signal transduction routes as mentioned hereinbefore (receptors which are used by the factor, or signal transduction routes which are activated by the factor) can lead to identification of new targets for diagnostics and/or treatment in the brain disorders referred to above.

The invention will presently be further elucidated in and by the following example.

### EXAMPLE

Within our investigation, we study populations of purified astrocytes, oligodendrocytes and microglia from adult human brains, which are obtained after speedy necropsy. Necropsy as a rule takes place within six hours of death. Donors of this material have given explicit permission during their life for taking brain material. During our studies it was observed that directly after addition of a cell-free and water soluble extract of human white matter (the area in the central nervous system where many nerve tracts run but where no neurons are located) to cell suspensions coming from the gray matter (the area where cortical neurons themselves are located), a cell population started to grow that exhibited all characteristics of adult neurons. This effect was not seen, or hardly so, in cell suspensions of gray matter cells to which such a white matter extract was not added. The growing neurons were further purified to separate them from other types of neural cells (astrocytes, oligodendrocytes and microglia), and thus cultures were obtained in which neurons represent at least 95% of all cells. Addition of the white matter extract to this purified population again yielded immediate cell division, something that was not seen in untreated cultures. After 2-3 weeks, the neurons in untreated cultures die, while in the cultures to which the extract is added every week, the neurons survive and increase in number every time directly after the addition of the extract. These neuron cultures can be continued up to at least 60 days after the start of the culture. About a month after the start of the culture, the neurons further start to exhibit a certain amount of regeneration of dendritic processes. However, this effect is limited and large-scale regeneration of axons or dendrites does not seem to take place as yet.

The active constituents of the extract, which lead to division of neurons, are different from the growth factors described earlier. As has already been found by others too, addition of the growth factors NGF, EGF and FGF-2 does not lead to a division of neurons under culture conditions, while addition of the white matter extract does stimulate the neurons to divide.

Hereinbelow it will be described what isolation and culturing method for neurons was employed, what criteria were used to identify the cells as neurons, what criteria were used to establish that cell division is involved, and what the method of preparation of the white matter extract is. The observations were made with post-mortem neurons from the cerebral cortex of at least five different donors, none of whom - according to information of the Nederlandse Hersenbank in Amsterdam - suffered from a recognizable brain disorder. Neurons coming from deceased patients with Alzheimer's disease or multiple sclerosis exhibit a similar behavior to control neurons and also proceed to divide after addition of the extract.

### Purification and culture of neurons from human post-mortem brain tissue

Directly after necropsy, the gray matter from human brains is stored in Dulbecco's Modified Eagle Medium (DMEM)/HAMF-10 provided with 50 microgram/mL of gentamicin. The gray matter can be successfully worked up within 24 hours of necropsy. Portions of 3 g of gray matter are fine-cut in 2 mL digestion medium (= 0.25% (w/w/) trypsin and 0.1 mg/mL DNAse) and incubated at 37°C for 20 minutes. The supernatant is removed and 20 mL of culture medium is added (culture medium = DMEM/HAMF-10 provided with 10% fetal calf serum, 2 mM L-glutamine, 100 U/mL penicillin and 0.1 mg/mL streptomycin). The cell mass is washed twice by centrifugation at 500 g for 9 minutes at 20°C and resuspension in culture medium. Thereupon the cell mass is included in 20 mL culture medium and passed through a 100 micron-filter.

After this, neurons (and other cells) are separated as far as possible from myelin membranes which are co-obtained from the tissue and are still present in the cell suspension as small fatty particles. The cells with myelin residues therein are again collected by centrifugation and subsequently included in a mixture of 14 mL Percoll-solution, 1.3 mL 1.5 M NaCl and 40 mL gradient buffer (= 20 mM Na₂HPO₄; 5.6 mM NaH₂PO₄, 137 mM NaCl, 5.4 mM KCl, 2 g/L glucose and 0.2% bovine serum albumin, pH 7.4). The density of this mixture is 1.03 g/L. On top of the suspension thus formed, another 6 mL of the gradient buffer are carefully added, and then the whole is centrifuged at 1,250 g at 20° C for 30 min, without using the brake of the centrifuge.

Residues of myelin chiefly collect in the supernatant layer; it is carefully removed and the pellet and interphase with the neurons therein is washed with culture medium as describe hereinabove. After collecting the cell pellet after a final washing, the pellet is suspended in 5 mL lysis solution (= 6 M NH₄Cl and 0.3 mM KHCO₃) and the whole is incubated on ice for 10 to 20 min. The red blood cells present as contaminant thereby lyse.

Thereafter, the cells (mainly neurons, astrocytes and microglia), collected by centrifuging for 10 min at 500 g and at 4°C, are suspended in 10 mL culture medium and stored for 4 to 7 days in an uncoated culture flask of an area of 75 cm² under standard culture conditions (37°C, moisturized atmosphere, 5% CO₂ in the atmosphere). Adherent cells such as astrocytes and microglia can thus be separated from the non-adherent neurons. The neurons that remain present in suspension are collected from the culture medium by centrifugation for 10 min at 500 g at 20°C and resuspended in 15 mL culture medium. Amounts of 1.5 mL of this suspension are put in separate wells of a 12-well culture plate (culturing area per well about 3.8 cm²).

Addition of the white matter extract (about 2 mg/mL protein; see below) is done by diluting the extract 20-500 times in medium. Fresh portions of the extract are added every week in the same final dilution.

### Criteria for identification of neurons

The cells that represent more than 95% of the population in the culture, and that divide, were characterized on the basis of the following criteria:

### 1. Morphology

The comparatively large cells have a pyramidal or more rounded shape, often with residues of dendritic processes (which have largely been broken down by the isolation process). The cells further contain clearly observable inclusions, so-called lipofuscin vesicles, which are characteristic of adult neurons. The cells as a rule do not adhere, but exhibit minor adherence to the bottom of the culture vessel when the culture medium contains relatively many myelin membranes. Residues of small myelin fragments, left behind during the isolation procedure (although it is aimed at removing myelin as far as possible), can lead to a situation where also in cultures to which no white matter extract is added, still in the first phases of culturing, a certain amount of neuronal division occurs. On morphological grounds, neurons are well distinguishable from astrocytes, oligodendrocytes or microglia.

### 2. Immunohistochemical markers

The cells contain without exception the following neuronal markers (e.g. Black et al, 1992), such as established through immunocytochemical methods: neuron-specific enolase (NSE) and the neurofilaments NE14, NE68 and NE160. The presence of lipofuscin vesicles is also regarded as a typical marker for adult neurons.
None of the cells contain the markers glial fibrillary acidic protein (GFAP; a marker for astrocytes), myelin oligodendrocyte glycoprotein (MOG) and alpha B-crystalline (both markers for oligodendrocytes), or CD68 (a marker for microglia/macrophages).
All the neurons are also negative for the intermediate filament protein nestin, a protein occurring inter alia in neuronal precursor cells. This indicates that the neurons are indeed adult, differentiated neurons and not undifferentiated neuronal precursor cells.

### 3. Markers of gene expression

By means of the polymerase chain reaction (PCR) it was established that the purified population of neurons (more than 95% homogeneity based on morphological and immunocytochemical criteria) express the same marker genes as those detected through immunocytochemical techniques.

### 4. Electrophysiological characteristics

The neurons which after division were maintained in culture for at least three weeks, were examined for electrophysiological functions using patch-clamp electrophysiology. Because medium with 10% serum is used, neurons do not adhere and exhibit axonal or dendritie processes only to a very limited extent. Corresponding with the little differentiated morphology of the neurons, the cells exhibit a limited activity of K⁺ channels and no clear activity of Na⁺ or Ca²⁺ channels. This observation indicates that the neurons under the conditions employed traverse a certain extent of de-differentiation; this condition is possibly necessary to enable the cells to proceed to divide.

### Criteria for establishing cell division

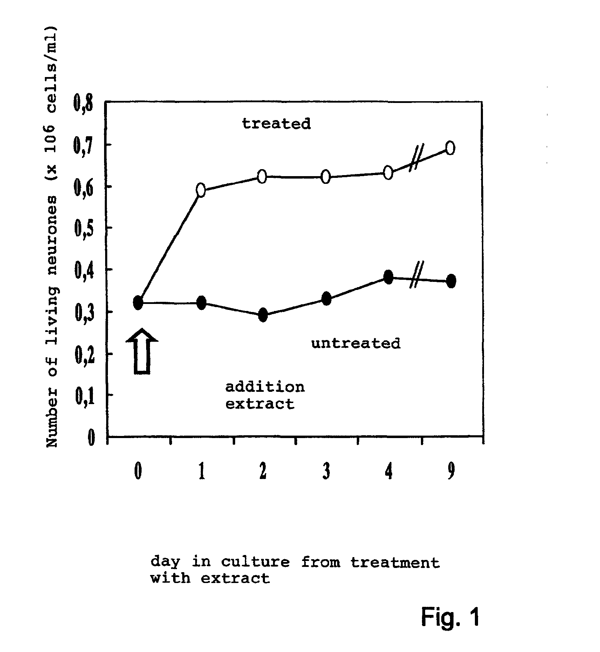

That the neurons are made to divide after addition of the white matter extract was established as follows:
1. Within 24 hours of addition of the extract, the total number of viable neurons in the culture doubled, as was measured with standard cell counting methods. The count is limited to only those cells that clearly exhibit the morphology of neurons, including the possession of lipofuscin vesicles. In the counts, further, only those cells were included that exclude propidium iodide (5 microgram/mL), as a sign of good viability. Dead cells take up propidium iodide and under blue excitation a dead cell can be recognized, with a fluorescence microscope, by red fluorescence. In untreated cultures of neurons the number of neurons remains constant over the same period. Fig. 1 gives an example of such a measurement for adult neurons from normal brains. The cell increase was established repeatedly.
2. Within 24 or 48 hours after addition of the white matter extract, and in the presence of 5'-bromo-2'-deoxyuridine (BrdU), to neurons in culture, accumulation of BrdU in the nuclei of neurons can be demonstrated. This happens only when DNA replication occurs and the labeled BrdU is incorporated into the new synthesized DNA. BrdU labeling is therefore a classic method of demonstrating DNA replication, and hence cell division. By means of immunofluorescence methods, BrdU can be found in the nucleus of cells which both contain the lipofuscin vesicles (strongly autofluorescent cell constituents) and express the typical neuronal marker NSE, i.e. neurons.

### Nature of the white matter extract

The extract was prepared from starting material that was formed by white matter samples from 11 different donors of the Nederlandse Hersenbank, none of whom, as far as was known, suffered from any brain disorder. The white matter was taken from the cerebrum by a neuropathologist, as a rule within about six hours of the death of the donor. Macroscopically, the samples did not exhibit any deviations and they consisted completely of white matter. After being taken, the samples were frozen immediately in liquid nitrogen, and then stored at -80°C.

To prepare the extract, white matter samples from 11 different donors were put together, provided with a small amount of phosphate-buffered physiological salt solution (PBS, pH 7.4) and, after defrosting, homogenized by means of a so-called Dounce homogenizer to form a milky suspension. In this way, 12 grams of tissue were homogenized to a suspension of a total volume of 50 mL. To prepare an extract, the suspension was centrifuged at 10,000 g at a temperature of 4 °C for 10 minutes. The supernatant was removed (about half of the volume) and the pellet in turn was included in PBS and homogenized by means of the Dounce homogenizer. After renewed centrifugation as indicated hereinabove, the second supernatant was added to the first. The total volume of the supernatant that is thus obtained is equal to the total volume of the original suspension, i.e. 50 mL. The thus obtained clear extract was dialyzed overnight in standard dialysis tubing (molecular weight cut-off about 3,000 Da) against 1.5 L PBS at a temperature of 4 °C. The dialyzed supernatant was stored in small portions at -20°C. The total protein concentration in it is 2 mg/mL. To obtain the effect described, the white matter extract is added to the culture medium, such that eventually it is diluted 20-500x.

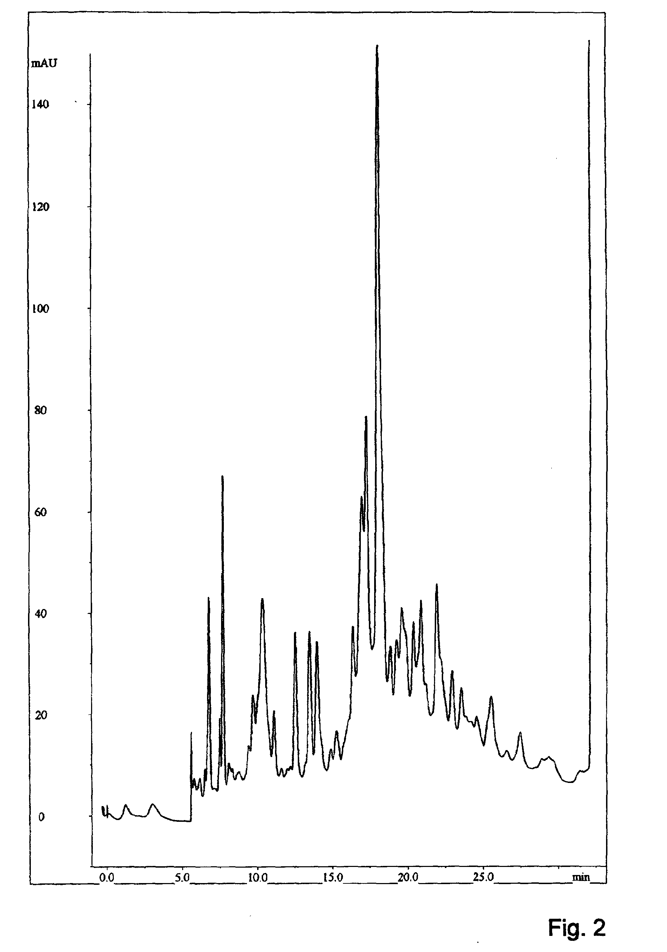

The extract thus prepared contains a large number of different water soluble, high-molecular compounds, typically proteins. Some insight into the complexity of the extract can be derived from a reversed-phase high-performance liquid chromatography (RP-HPLC) profile of the extract (appended as Fig. 2). From this profile, it can be derived that a very large number of different substances are present in the extract. It is not known yet whether the extract contains a single active substance or whether perhaps a combination of different substances is required to stimulate the cell division of neurons.

### References

- Barish ME, Mansdorf NB and Raissdana SS (1991) Gamma-interferon promotes differentiation of cultured cortical and hippocampal neurons. Dev. Biol. 144, 412-423.
- Black RS, Bouvier MM, Rex Sheu K-W, Darzynkiewicz Z and Blass JP (1992) Presence of typical neuronal markers in serially cultured cells from adult human brain. J. Neurol. Sci. 111, 104-112.
- Brewer GJ (1997) Isolation and culture of adult rat hippocampal neurons. J. Neurosci. Meth. 71, 143-155.
- Brewer GJ (1999) Regeneration and proliferation of embryonic and adult rat hippocampal neurons in culture. Exp. Neurol. 159, 237-247.
- Cataneo E and McKay R (1990) Proliferation and differentiation of neuronal stem cells regulated by nerve growth factor. Nature 347, 762-765.
- DeLima AD and Vogt T (1999) Astroglia inhibit the proliferation of neocortical cells and prevent the generation of small GABAergic neurons in vitro. Eur. J. Neurosci. 11, 3845-3856.
- Eriksson PS, Perfliveva E, Bjork-Eriksson T, Alborn AM, Nordborg C, Peterson DA, Gage FH (1998) Neurogenesis in the adult human hippocampus. Nat. Med. 4, 1313-1317.
- Gage FH, Coates PW, Palmer TD, Kuhn HG, Fischer LJ, Suhonen JO, Peterson DA, Suhr ST and Ray J (1995) Survival and differentiation of adult neuronal progenitor cells transplanted to the adult brain. Proc. Natl. Acad. Sci. USA 92, 11879-11883.
- Gould E, Reeves AJ, Graziano MSA and Gross CG (1999) Neurogenesis in the neocortex of adult primates. Science 286, 548-552.
- Kalyani AJ, Piper D, Mujtaba T, Lucero MT and Roa MS (1998) Spinal cord neuronal precursors generate multiple neuronal phenotypes in culture. J. Neurosci. 18, 7856-7868.
- Kirschenbaum B and Goldman SA (1995) Brain-derived neurotrophic factor promotes the survival of neurons arising from the adult rat forebrain subependymal zone. Proc. Natl. Acad. Sci USA 92, 210-214.
- Kornack DR and Rakic P (1999) Continuation of neurogenesis in the hippocampus of the adult macaque monkey. Proc. Natl. Acad. Sci. USA 96, 5769-5773.
- Kuhn HG, Winkler J, Kempermann G, Thal LJ and Gage FH (1997) Epidermal growth factor and fibroblast growth factor-2 have different effects on neural progenitors in the adult rat brain. J. Neurosci. 17, 5820-5829.
- Lois C and Alvarex-Buylla A (1994) Long-distance neuronal migration in the adult mammalian brain. Science 264, 1145-1148.
- Morrison RS, Sharma A, the Vellis J and Bradshaw RA (1986) Basic fibroblast growth factor supports the survival of cerebral cortical neurons in primary culture. Proc. Natl. Acada. Sci. USA 83, 7537-7541.
- Morrison RS, Kornblum HI, Leslie FM, Bradshaw RA (1987) Trophic stimulation of cultured neurons from neonatal rat brain by epidermal growth factor. Science 238, 72-75.
- Mrak RE, Griffin ST and Graham DI (1997) Aging-associated changes in human brain. J. Neuropathol. Exp. Neurol. 56, 1269-1275.
- Niederöst BP, Zimmermann DR, Schwab, ME and Bandtlow CE (1999) Bovine CNS myelin contains neurite growth-inhibitory activity associated with chondroitin sulfate proteolglycans. J. Neurosci. 19, 8979-8989.
- O'Leary (1993) Adding neurons to the adult mammalian brain (commentary). Proc. Natl Acad. Sci. USA 90, 2101-2102.
- Palmer TD, Markakis EA, Willhoite AR, Safar F and Gage FH (1999) Fibroblast growth factor-2 activates a latent neurogenic program in neural stem cells from diverse regions of the adult CNS. J. Neurosci. 19, 8487-8497.
- Ray JR, Peterson DA, Schinstine M and Gage FH (1993) Proliferation, differentiation and long-term culture of primary hippocampal neurons. Proc. Natl. Acad. Sci. USA 90, 3602-3606.
- Reynolds BA and Weiss S (1992) Generation of neurons and astrocytes from isolated cells of the adult mammalian central nervous system. Science 255, 1707-1710.
- Richards LJ, Kilpatrick TJ and Bartlett PF (1992) De novo generation of neuronal cells from the adult mouse brain. Proc. Natl. Acad. Sci. USA 89, 8591-8595.
- Schnell L and Schwab ME (1990) Axonal regeneration in the rat spinal cord produced by an antibody against myelin-associated neurite growth inhibitors. Nature 343, 269-272.
- Schwab ME (1996) Molecules inhibiting neurite growth: a minireview. Neurochem. Res. 21, 755-761.
- Unsicker K, Reichert-Preibisch H, Schmidt R, Pettmann B, Labourdette G and Sensenbrenner M (1987) Astroglial and fibroblast growth factors have neurotrophic functions for cultured peripheral and central nervous system neurons. Proc. Natl. Acad. Sci. USA 84, 5459-5463.
- Vescovi AL, Snyder EY (1999) Establishment and properties of neural stem cell clones: plasticity in vitro and in vivo. Brain Pathol. 9, 569-598.
- Walicke P, Cowan WM, Ueno N, Baird A and Guillemin R (1986) Fibroblast growth factor promotes survival of dissociated hippocampal neurons and enhances neurite extension. Proc. Natl. Acad. Sci. USA 83, 3012-3016.
- Whitson JS, Selkoe DJ and Cotman CW (1989) Amyloid-b protein enhances the survival of hippocampal neurons in vitro. Science 243, 1488-1490.
- Zhu X, Raina AK and Smith MA (1999) Cell cycle events in neurons (commentary). Am. J. Pathol. 155, 327-329.

## Claims

1. A method for obtaining a composition or factor which is capable of stimulating neurons to cell division, wherein the composition or factor is recovered from white matter of the brain or tissue of the central nervous system.

2. A method according to claim 1, wherein the brain or the tissue of the central nervous system originate from a mammal, preferably a human.

3. A method according to claim 1 or 2, wherein an extract of white matter of the brain or white matter of tissue of the central nervous system is made.

4. A method according to claim 3, wherein the extract is prepared by homogenizing white matter of the brain or of tissue of the central nervous system, centrifuging homogenized material, and recovering the liquid.

5. A method according to claim 3 or 4, wherein work is done at a temperature below 10°C.

6. A method according to any one of the preceding claims 3-5, wherein the white matter is included in a suitable buffer.

7. A method according to claim 6, wherein the buffer is a phosphate-buffered physiological salt solution having a pH of 6.5-8.5, preferably of 7-8.

8. A method according to one or more of claims 3-7, wherein the extract is subjected to purification and/or fractionation and a composition or factor is recovered which possesses the capacity to stimulate neurons to cell division.

9. A method according to claim 8, wherein the extract is dialyzed against a buffer consisting of a phosphate-buffered physiological salt solution having a pH of 6.5-8.5, preferably of 7-8.

10. A method according to claim 9, wherein the dialysis is carried out such that substances of a molecular weight of less than 3,000 are removed.

11. A composition or factor having the capacity to stimulate neurons to cell division, obtainable by the method according to one or more of claims 1-10.

12. A method for stimulating neurons to cell division, wherein neurons are contacted with a composition or factor according to claim 11.

13. A method according to claim 12, which is carried out in vitro for culturing and/or producing neurons.

14. A method according to claim 13, wherein the neurons originate from a mammal, preferably a human.

15. A method according to claim 12, which is carried out in vivo in a mammal, preferably human, with a brain disorder involving disturbed cell division of neurons.

16. A method according to claim 15, wherein the contact between the composition or factor with the neurons is realized by administering the composition or factor to the mammal in the form of a pharmaceutical preparation.

17. A pharmaceutical preparation comprising a composition or factor according to claim 11, as well as a pharmaceutically acceptable carrier.
